# EUROPÄISCHE PATENTSCHRIFT

(11) **EP 0 440 846 B1**
(45) Veröffentlichungstag und Bekanntmachung des Hinweises auf die Patenterteilung: **09.06.1993**
(21) Anmeldenummer: 90102373.9
(22) Anmeldetag: 07.02.1990
(51) Int. Cl.: A61M 5/315

(54) **Doppelkammerspritze und Verwendungsverfahren**
Two-compartment syringe and application process
Seringue à chambre double et procédure d'emploi

(43) Veröffentlichungstag der Anmeldung: 14.08.1991
(73) Patentinhaber: Arzneimittel GmbH Apotheker Vetter & Co. Ravensburg, D-88212 Ravensburg (DE)
(72) Erfinder: Vetter, Udo J., D-7980 Ravensburg (DE)
(74) Vertreter: Dziewior, Joachim, Dipl.-Phys. Dr.

(56) Entgegenhaltungen:
- EP-A- 0 328 699
- GB-A- 1 214 053

## Beschreibung

Die Erfindung betrifft ein Verfahren zur Durchmischung einer in einer Doppelkammerspritze abgefüllten pharmazeutischen Substanz, deren Wirkstoff sich in trockener Form in der einen, nadelseitigen Kammer und deren Lösungsmittel sich in der anderen Kammer befindet, wobei die beiden Kammern durch einen Stopfen voneinander abgetrennt sind, der zur Durchmischung der Substanz mit dem Lösungsmittel vor der Applikation in den Bereich eines im Spritzenzylinder vorgesehenen, sich in dessen axialer Richtung erstreckenden By-passes verstellbar ist, dessen Länge größer als die axiale Höhe des Stopfens ist, wobei der Stopfen zum nadelseitigen Ende hin über den die zweite Kammer begrenzenden, mit einer Kolbenstange versehenen Spritzenkolben direkt oder über das Lösungsmittel verschoben wird. Ferner betrifft die Erfindung eine Spritze zur Durchführung des Verfahrens.

Aus der europ. Patentanmeldung EP-A-0 328 699 ist eine Doppelkammerspritze bekannt, die sicherstellt, daß das Lösungsmittel aus der einen Kammer mit begrenzter Geschwindigkeit in die andere, den Wirkstoff in trockener Form enthaltende Kammer überführt werden kann. Hierdurch wird gewährleistet, daß Teile des Lösungsmittels bzw. des Wirkstoffes nicht bereits vorab infolge zu hoher Strömungsgeschwindigkeit des Lösungsmittels aus dem Spritzenansatz austreten können. Diese Spritze hat sich insbes. für leicht lösliche Wirkstoffe in der Praxis bewährt. Bei schwerer löslichen Wirkstoffen muß dagegen dafür gesorgt werden, daß vor der Applikation der Spritze eine vollständige Auflösung des Wirkstoffes stattgefunden hat.

Der Erfindung liegt die Aufgabe zugrunde, ein Verfahren der o. g. Art sowie eine Spritze zur Durchführung des Verfahrens so zu verbessern, daß die Möglichkeit besteht, auch schwer lösliche Substanzen und Wirkstoffe in möglichst kulzer Zeit auf einfache und zuverlässige Weise in dem Lösungsmittel in Lösung zu bringen.

Ein diese Aufgabe lösendes Verfahren ist dadurch gekennzeichnet, daß bei vertikaler Anordnung mit nach oben weisendem Nadelansatzende zunächst der Stopfen mit vorrichtungsmäßig begrenzter Geschwindigkeit in den Bereich des By-passes verstellt wird, daß dann der Spritzenkolben durch freie Verschiebbarkeit der Kolbenstange gegen einen vorderen Anschlag bis zur Anlage an den Stopfen gebracht und dadurch das Lösungsmittel in die nadelseitige Kammer überführt wird, das anschließend bis zur vollständigen Auflösung der Substanz der Spritzenkolben einmal oder mehrfach bis zu einem hinteren Anschlag der Kolbenstange wieder zurückgezogen und danach erneut zum vorderen Anschlag verstellt wird, so daß der Wirkstoff mit dem Lösungsmittel von der vorderen Kammer über den By-pass in die hintere Kammer und anschließend in die vordere Kammer wieder zurückströmt, und daß schließlich nach vollständiger Auflösung des Wirkstoffs der die Verschiebung des Spritzenkolbens bis zum nadelseitigen Ende des Spritzenzylinders hin zunächst begrenzende vordere Anschlag der Kolbenstange zur Applikation der pharmazeutischen Substanz gelöst wird.

Der durch die Erfindung erreichte Fortschritt besteht im wesentlichen darin, daß die vollständige Auflösung des Wirkstoffes durch eine sehr einfache Handhabung der Spritze gewährleistet ist. Je nach Löslichkeit der Substanz kann dabei vorgeschrieben werden, wie oft der Spritzenkolben zwischen den beiden Anschlägen hin und herbewegt werden muß. Da das Lösungsmittel mit dem darin befindlichen Wirkstoff hierbei jedesmal durch den By-pass strömen muß, wird eine starke Durchmischung beider Bestandteile erreicht. Dabei sorgen die beiden Anschläge einerseits dafür, daß der Spritzenkolben den Stopfen nicht über den Bereich des By-passes hinausschiebt bzw. dafür, daß der Spritzenkolben nicht versehentlich aus dem Spritzenzylinder endseitig herausgezogen wird.

In bevorzugter Ausführungsform der Erfindung ist weiter vorgesehen, daß die Kanüle bei gegen den hinteren Anschlag verstellter Kolbenstange angesetzt wird.

In vorrichtungsmäßiger Hinsicht betrifft die Erfindung zur Durchführung des oben beschriebenen Verfahrens eine Spritze für medizinische Zwecke, mit einem am einen Ende zum Ansatz einer Injektionsnadel ausgebildeten Spritzenzylinder und mit einem im Spritzenzylinder verschiebbaren Spritzenkolben sowie einem Stopfen, der zwischen dem Nadelansatzende und dem Spritzenkolben angeordnet ist und so zwei Kammern einer Zweikammerspritze bildet, wobei in der nadelseitigen Kammer die Mantelfläche des Spritzenzylinders mit einer sich axial erstreckenden, einen By-pass bildenden Querschnittserweiterung versehen ist, über die beide Kammern bei in den Bereich des By-passes verstelltem Stopfen miteinander verbunden sind, sowie mit einem als Kolbenbremse dienenden Dämpfungsteil am dem Nadelansatzende entgegengesetzten Zylinderende.

Eine solche, zur Durchführung des beschriebenen Verfahrens geeignete Spritze ist dadurch gekennzeichnet, daß das Dämpfungsteil die Verstellgeschwindigkeit der Kolbenstange begrenzt, bis der Stopfen in den Bereich des By-Passes verstellt ist und daß die Kolbenstange zur Begrenzung des Verstellhubs des Spritzenkolbens zwischen seiner Ausgangsstellung am Zylinderende und seiner Anlage an dem in den By-pass-Bereich verstellten Stopfen einen vorderen und einen hinteren Anschlag für ein am Zylinderende vorgesehenes Gegenstück aufweist, und daß der vordere, die Verstellung des Spritzenkolbens zum nadelseitigen Ende hin begrenzende Anschlag lösbar ist.

Das Dämpfungsteil verhindert nicht nur ein versehentliches Betätigen der Kolbenstange, etwa noch im steril verpackten Zustand der Fertigspritze, sondern sorgt darüber hinaus dafür, daß der Stopfen nicht infolge einer zu hohen Verstellgeschwindigkeit der Kolbenstange über den By-pass-Bereich hinaus verschoben werden kann. Dies würde nämlich die spätere Durchmischung des Wirkstoffs mit dem Lösungsmittel über den By-pass hinweg verhindern. Nach erfolgter Auflösung des Wirkstoffes wird dann der vordere Anschlag für die Applikation der Spritze gelöst.

In bevorzugter Ausführungsform der Erfindung ist das Dämpfungsteil von einer Fingerauflage gebildet, die auf einem Glaswulst auf der dem Nadelansatzende abgewandten Seite des Spritzenzylinders aufgesetzt und mit einem zum Spritzenzylinder koaxialen Innengewinde für einen an der Kolbenstange angeordneten Gewindeschaft versehen ist, wobei das Innengewinde das Gegenstück und die dem Spritzenzylinder abgewandte Stirnseite den hinteren Anschlag bilden und die Länge des Gewindeschafts so bemessen ist, daß seine dem Spritzenkolben abgewandte Stirnseite aus dem Innengewinde freikommt, sobald der Stopfen sich im Mittelbereich des By-passes befindet.

Der vordere Anschlag ist zweckmäßigerweise von der dem ersten Gewindeschaft zugewandten Stirnfläche eines zweiten, ebenfalls zum Innengewinde korrespondierenden Gewindeschaftes gebildet. Auf diese Weise läßt sich der vordere Anschlag besonders einfach lösen, indem nämlich der zweite Gewindeschaft durch das Innengewinde hindurchgeschraubt wird.

Die Kolbenstange weist in vorteilhafter Weiterbildung der Erfindung stirnseitig einen Gewindeansatz für ein im Spritzenkolben vorgesehenes Stopfengewinde auf, wobei zwischen dem Gewindeansatz und dem ersten Gewindeschaft ein zylindrisches Führungsstück vorgesehen ist, dessen Durchmesser nur geringfügig kleiner als der Kerndurchmesser des Innengewindes ist. Auf diese Weise kann die Kolbenstange in den Zylinderkolben eingeschraubt werden, so daß dieser ohne weiteres über die Kolbenstange zurückgezogen werden kann. Das zylindrische Führungsstück erleichtert hierbei die Montage der Kolbenstange im Spritzenkolben.

Die Fingerauflage ist zweckmäßigerweise von zwei Schalenhälften gebildet, die zwischen sich den Glaswulst des Spritzenzylinders aufnehmen und mit gegenseitig einrastenden Verbindungselementen versehen sind, wobei die kanülenseitige Schalenhälfte mit einer den Spritzenzylinder umschließenden Ausnehmung und die andere Schalenhälfte mit dem Innengewinde versehen ist. Dabei kann zur Vereinfachung der Montage vorgesehen sein, daß die beiden Schalenhälften randseitig gelenkig miteinander verbunden sind.

Im folgenden wird die Erfindung an einem in der Zeichnung dargestellten Ausführungsbeispiel näher erläutert; es zeigen:
- Fig. 1: einen Querschnitt durch eine Spritze in ihrer Ausgangsstellung,
- Fig. 2: eine der Fig. 1 entsprechende Darstellung, jedoch in der Gebrauchstellung zur Lösung des Wirkstoffs in dem Lösungsmittel,
- Fig. 3: den Gegenstand nach Fig. 1, jedoch in der für die Applikation des pharmazeutischen Wirkstoffs vorbereiteten Anwendungsstellung.

Die in der Zeichnung dargestellte Spritze für medizinische Zwecke besteht aus einem Spritzenzylinder 1, der an seinem einen Ende zum Ansatz einer Injektionsnadel 2 eingerichtet ist. Im Spritzenzylinder 1 ist ein verschiebbarer Spritzenkolben 3 sowie ein Stopfen 4 vorgesehen, der zwischen dem Nadelansatzende 5 und dem Spritzenkolben 3 angeordnet ist und so das Innere des Spritzenzylinders 1 in zwei Kammern 6, 7 einer Zweikammerspritze unterteilt.

In der nadelseitigen Kammer 6 ist die Mantelfläche des Spritzenzylinders 1 mit einer sich axial erstreckenden, einen By-pass 8 bildenden Querschnittserweiterung versehen. Über diesen By-pass 8 sind die beiden Kammern 6, 7 bei in den Bereich des By-passes 8 verstelltem Stopfen 4 miteinander verbunden.

An dem dem Nadelansatzende 5 entgegen gesetzten Zylinderende ist ein Dämpfungsteil 9 vorgesehen, das die Verstellgeschwindigkeit der Kolbenstange 10 begrenzt. Im einzelnen ist dieses Dämpfungsteil 9 von einer Fingerauflage 12 gebildet, die auf einem Glaswulst 11 auf der dem Nadelansatzende 5 abgewandten Seite des Spritzenzylinders 1 aufgesetzt ist. Die Fingerauflage 12 ist mit einem zum Spritzenzylinder 1 koaxialen Innengewinde versehen, das zu einem an der Kolbenstange 10 angeordneten Gewindeschaft 13 korrespondiert. Dabei ist die Länge dem Gewindeschaftes 13 so bemessen, daß durch das Hindurchschrauben dieses Gewindeschaftes 13 durch das Innengewinde der Stopfen 4 gerade in den Bereich des By-passes 8 verstellt wird.

Mit Abstand von dem ersten Gewindeschaft 13 weist die Kolbenstange 10 einen zweiten Gewindeschaft 14 auf, der ebenfalls zu dem Innengewinde der Fingerauflage 12 korrespondiert. Die beiden einander zugewandten Stirnflächen 13.1, 14.1 des ersten bzw. des zweiten Gewindeschaftes 13, 14 bilden jeweils einen vorderen bzw. hinteren Anschlag an dem Innengewinde, erlauben also eine Hin- und Herbewegung der Kolbenstange 10 innerhalb des durch die beiden Anschläge festgelegten Verstellhubes. Hierdurch besteht die Möglichkeit, das Lösungsmittel bei dem ersten Verstellhub in Richtung zum nadelseitigen Ende hin in die den Wirkstoff enthaltende Kammer 6 zu überführen und durch anschließendes Zurückziehen der Kolbenstange 10 den Wirkstoff zusammen mit dem Lösungsmittel über den By-pass 8 in die andere Kammer 7 zu überführen. Durch das u. U. mehrfache Überführen der Injektionslösung über den By-pass 8 wird eine relativ schnelle und zuverlässige Auflösung auch solcher Wirkstoffe und Substanzen erreicht, die sich in dem Lösungsmittel vergleichsweise schlecht lösen.

Die beiderseitigen Anschläge stellen dabei einerseits sicher, daß der Stopfen 4 nicht auf dem Bereich des By-passes 8 herausgedrückt werden kann und gewährleisten andererseits, daß die Kolbenstange 10 mit dem Spritzenkolben 3 nicht versehentlich aus dem Spritzenzylinder 1 herausgezogen werden kann.

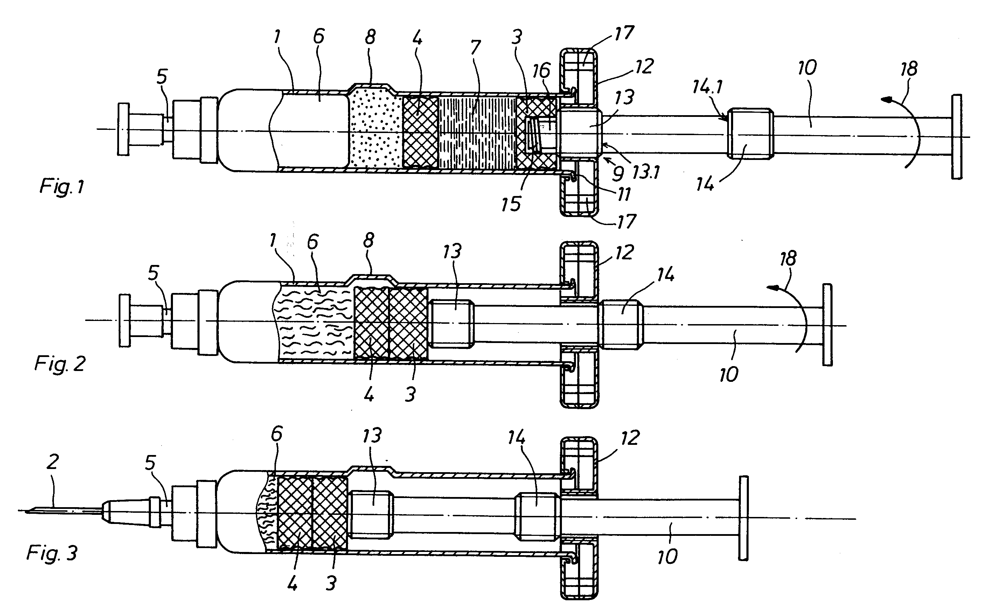

Nach vollständiger Auflösung des Wirkstoffs in dem Lösungsmittel wird bei zurückgezogenem Spritzenkolben 3 die Kanüle 2 auf das Spritzenansatzende 5 aufgesetzt, anschließend die Kolbenstange 10 gegen den vorderen Anschlag 14.1 verstellt und sodann der zweite Gewindeschaft 14 durch das Innengewinde in der durch den Pfeil 18 angedeuteten Weise hindurchgedreht, worauf sich die Spritze in der in Fig. 3 dargestellten, für die Applikation vorbereiteten Stellung befindet.

Zur Erleichterung der Montage der Kolbenstange 10 weist diese stirnseitig einen Gewindeansatz 15 für ein im Spritzenkolben 3 vorgesehenes Stopfengewinde auf, wobei zwischen dem Gewindeansatz 15 und dem ersten Gewindeschaft 13 ein zylindrisches Führungsstück 16 vorgesehen ist, dessen Durchmesser nur geringfügig kleiner als der Kerndurchmesser des Innengewindes ist.

Die Fingerauflage 12 ist von zwei Schalenhälften gebildet, die zwischen sich den Glaswulst 11 des Spritzenzylinders 1 aufnehmen. Die beiden Schalenhälften sind jeweils mit gegenseitig einrastenden, in der Zeichnung nur angedeuteten Verbindungselementen 17 versehen, wobei die kanülenseitige Schalenhälfte mit einer den Spritzenzylinder 1 umschließenden Ausnehmung und die andere Schalenhälfte mit dem Innengewinde versehen ist. Hierdurch besteht auch die Möglichkeit, die Fingerauflage 12 erst kurz vor der Vorbereitung und Anwendung der Fertigspritze an dieser anzubringen. Eine weitere Vereinfachung der Handhabung läßt sich dadurch erreichen, daß die beiden Schalenhälften randseitig gelenkig miteinander verbunden sind.

## Patentansprüche

1. Verfahren zur Durchmischung einer in einer Doppelkammerspritze abgefüllten pharmazeutischen Substanz, deren Wirkstoff sich in trockener Form in der einen, nadelseitigen Kammer (6) und deren Lösungsmittel sich in der anderen Kammer (7) befindet, wobei die beiden Kammern (6, 7) durch einen Stopfen (4) voneinander abgetrennt sind, der zur Durchmischung der Substanz mit dem Lösungsmittel vor der Applikation in den Bereich eines im Spritzenzylinder (1) vorgesehenen, sich in dessen axialer Richtung erstreckenden By-passes (8) verstellbar ist, dessen Länge größer als die axiale Höhe des Stopfens (4) ist, wobei der Stopfen (4) zum nadelseitigen Ende hin über den die zweite Kammer (7) begrenzenden, mit einer Kolbenstange (10) versehenen Spritzenkolben (3) direkt oder über das Lösungsmittel verschoben wird, dadurch gekennzeichnet, daß bei vertikaler Anordnung mit nach oben weisendem Nadelansatzende (5) zunächst der Stopfen (4) mit vorrichtungsmäßig begrenzter Geschwindigkeit in den Bereich des By-passes (8) verstellt wird, daß dann der Spritzenkolben (3) durch freie Verschiebbarkeit der Kolbenstange (10) gegen einen vorderen Anschlag (14.1) bis zur Anlage an den Stopfen (4) gebracht und dadurch das Lösungsmittel in die nadelseitige Kammer (6) überführt wird, daß anschließend bis zur vollständigen Auflösung der Substanz der Spritzenkolben 3 einmal oder mehrfach bis zu einem hinteren Anschlag (13.1) der Kolbenstange (10) wieder zurückgezogen und danach erneut zum vorderen Anschlag (14.1) verstellt wird, so daß der Wirkstoff mit dem Lösungsmittel von der vorderen Kammer (6) über den By-pass (8) in die hintere Kammer (7) und anschließend in die vordere Kammer (6) wieder zurückströmt, und daß schließlich nach vollständiger Auflösung des Wirkstoffs der die Verschiebung des Spritzenkolbens (3) bis zum nadelseitigen Ende des Spritzenzylinders (1) hin zunächst begrenzende vordere Anschlag (14.1) der Kolbenstange (10) zur Applikation der pharmazeutischen Substanz gelöst wird.

2. Verfahren nach Anspruch 1, dadurch gekennzeichnet, daß die Kanüle (2) bei gegen den hinteren Anschlag (13.1) verstellter Kolbenstange (10) angesetzt wird.

3. Spritze für medizinische Zwecke zur Durchführung des Verfahrens nach den Ansprüchen 1 und 2, mit einem am einen Ende zum Ansatz einer Injektionsnadel (2) ausgebildeten Spritzenzylinder (1) und mit einem im Spritzenzylinder (1) verschiebbaren Spritzenkolben (3) sowie einem Stopfen (4), der zwischen dem Nadelansatzende (5) und dem Spritzenkolben (3) angeordnet ist und so zwei Kammern (6, 7) einer Zweikammerspritze bildet, wobei in der nadelseitigen Kammer (6) die Mantelfläche des Spritzenzylinders (1) mit einer sich axial erstreckenden, einen By-pass (8) bildenden Querschnittserweiterung versehen ist, über die beide Kammern (6, 7) bei in den Bereich (8) des By-passes (4) verstelltem Stopfen miteinander verbunden sind, sowie mit einem als Kolbenbremse dienenden Dämpfungsteil (9) am dem Nadelansatzende (5) entgegengesetzten Zylinderende, dadurch gekennzeichnet, daß das Dämpfungsteil (9) die Verstellgeschwindigkeit der Kolbenstange (10) begrenzt, bis der Stopfen (4) in den Bereich des By-passes (8) verstellt ist und daß die Kolbenstange (10) zur Begrenzung des Verstellhubs des Spritzenkolbens (3) zwischen seiner Ausgangsstellung am Zylinderende und seiner Anlage an dem in den By-pass-Bereich verstellten Stopfen (4) einen vorderen (14.1) und einen hinteren (13.1) Anschlag für ein am Zylinderende vorgesehenes Gegenstück aufweist, und daß der vordere, die Verstellung des Spritzenkolbens (3) zum nadelseitigen Ende hin begrenzende Anschlag (14.1) lösbar ist.

4. Spritze nach Anspruch 3, dadurch gekennzeichnet, daß das Dämpfungsteil (9) von einer Fingerauflage (12) gebildet ist, die auf einem Glaswulst (11) auf der dem Nadelansatzende (5) abgewandten Seite des Spritzenzylinders (1) aufgesetzt und mit einem zum Spritzenzylinder (1) koaxialen Innengewinde für einen an der Kolbenstange (10) angeordneten Gewindeschaft (13) versehen ist, wobei das Innengewinde das Gegenstück und die dem Spritzenzylinder abgewandte Stirnseite den hinteren Anschlag bilden und die Länge des Gewindeschafts (13) so bemessen ist, daß seine dem Spritzenkolben (3) abgewandte Stirnseite aus dem Innengewinde freikommt, sobald der Stopfen (4) sich im Mittelbereich des By-passes (8) befindet.

5. Spritze nach Anspruch 4, dadurch gekennzeichnet, daß der vordere Anschlag (14.1) von der dem ersten Gewindeschaft (13) zugewandten Stirnfläche eines zweiten, ebenfalls zum Innengewinde korrespondierenden Gewindeschaftes (14) gebildet ist.

6. Spritze nach Anspruch 4 oder 5, dadurch gekennzeichnet, daß die Kolbenstange (10) stirnseitig einen Gewindeansatz (15) für ein im Spritzenkolben (3) vorgesehenes Stopfengewinde aufweist, wobei zwischen dem Gewindeansatz (15) und dem ersten Gewindeschaft (13) ein zylindrisches Führungsstück (16) vorgesehen ist, dessen Durchmesser nur geringfügig kleiner als der Kerndurchmesser des Innengewindes ist.

7. Spritze nach den Ansprüchen 4 bis 6, dadurch gekennzeichnet, daß die Fingerauflage (12) von zwei Schalenhälften gebildet ist, die zwischen sich den Glaswulst (11) des Spritzenzylinders (1) aufnehmen und mit gegenseitig einrastenden Verbindungselementen (17) versehen sind, wobei die kanülenseitige Schalenhälfte mit einer den Spritzenzylinder (1) umschließenden Ausnehmung und die andere Schalenhälfte mit dem Innengewinde versehen ist.

8. Spritze nach Anspruch 7, dadurch gekennzeichnet, daß die beiden Schalenhälften randseitig gelenkig miteinander verbunden sind.

## Claims

1. A method of intermixing a pharmaceutical substance which is contained in a double-chamber syringe and rose active material is disposed in dry form in the one chamber (6) which is towards the needle, and whose solvent is disposed in the other chamber (7), wherein the two chambers (6, 7) are separated from each other by a plug (4) which for intermixing of the substance with the solvent prior to application is displaceable into the region of a by-pass (8) which is provided in the syringe cylinder (1) and which extends in the axial direction thereof and whose length is greater than the axial dimension of the plug (4), wherein the plug (4) is displaced towards the needle end by way of the syringe plunger (3) which delimits the second chamber (7) and which is provided with a plunger rod (10), directly or by way of the solvent, characterised in that when the syringe is arranged vertically with the needle attachment end (5) pointing upwardly, firstly the plug (4) is displaced into the region of the by-pass (8) at a speed which is limited by apparatus considerations, that then the syringe plunger (3) is put into a condition of bearing against the plug (4) by virtue of free displaceability of the plunger rod (10) towards a front stop (14.1) and thereby the solvent is transferred into the chamber (6) at the needle side, that then the syringe plunger (3) is withdrawn again as far as a rear stop (13.1) on the plunger rod (10) and then displaced again to the front stop (14.1) so that the active material with the solvent flows back from the front chamber (6) by way of the by-pass (8) into the rear chamber (7) and then into the front chamber (6) again one or more times, until the substance is completely dissolved, and that finally after the active material is completely dissolved the front stop (14.1) on the plunger rod (10), which initially restricts the displacement of the syringe plunger (3) towards the needle end of the syringe cylinder (1) is released for application of the pharmaceutical substance.

2. A method according to claim 1 characterised in that the needle (2) is fitted when the plunger rod (10) is moved against the rear stop (13.1).

3. A syringe for medical purposes for carrying out the method according to claims 1 and 2 comprising a syringe cylinder (1) which is adapted at one end for attachment of an injection needle (2), and a syringe plunger (3) which is displaceable in the syringe cylinder (1), and a plug (4) which is disposed between the needle attachment end (5) and the syringe plunger (3) and thus forms two chambers (6, 7) of a double-chamber syringe, wherein in the chamber (6) which is towards the needle the peripheral surface of the syringe cylinder (1) is provided with an axially extending cross-sectional enlargement which forms a by-pass (8) and by way of which both chambers (6, 7) are communicated with each other when the plug is displaced into the region (8) of the by-pass (5), and further comprising a damping member (9) which serves as a plunger brake at the end of the cylinder which is opposite to the needle attachment end (5), characterised in that the damping member (9) restricts the speed of displacement of the plunger rod (10) until the plug (4) is displaced into the region of the by-pass (8) and that the plunger rod (10), for restricting the displacement stroke movement of the syringe plunger (3) between its starting position at the end of the cylinder and its condition of bearing against the plug (4) when displaced into the by-pass region, has a front stop (14.1) and a rear stop (13.1) for a co-operating portion provided at the end of the cylinder, and that the front stop (14.1) which restricts the displacement of the syringe plunger (3) towards the needle end is releasable.

4. A syringe according to claim 3 characterised in that the damping member (9) is formed with a finger support (12) which is fitted on a glass flange (11) on the side of the syringe cylinder (1) which is remote from the needle attachment end (5), and is provided with a female screwthread which is coaxial with respect to the syringe cylinder (1) for a screwthreaded shank (13) disposed on the plunger rod (10), wherein the female screwthread from the co-operating portion and the end face which is remote from the syringe cylinder forms the rear stop, and the length of the screwthreaded shank (13) is such that its end face which is remote from the syringe plunger (3) comes out of the female screwthread as soon as the plug (4) is in the middle region of the by-pass (8).

5. A syringe according to claim 4 characterised in that the front stop (14.1) is formed by the end face, which is towards the first screwthreaded shank (13), of a second screwthreaded shank (14) which also corresponds to the female screwthread.

6. A syringe according to claim 4 or claim 5 characterised in that the plunger rod (10) is provided at its end with a thread attachment (15) for a plug screwthread in the syringe plunger (3), wherein provided between the thread attachment (15) and the first screwthreaded shank (13) is a cylindrical guide portion (16) whose diameter is only slightly smaller than the minor thread diameter of the female screwthread.

7. A syringe according to claims 4 to 6 characterised in that the finger support (12) is formed by two shell halves which accommodate the glass flange (11) of the syringe cylinder (1) between them and which are provided with mutually engaging connecting elements (17), wherein the shell half which is towards the needle end is provided with an opening which embraces the syringe cylinder (1) and the other shell half is provided with the female screwthread.

8. A syringe according to claim 7 characterised in that the two shell halves are hingedly connected to each other at the edges.

## Revendications

1. Procédé pour mélanger de façon intime une substance pharmaceutique introduite dans une seringue à deux chambres et dont la substance active est située, à l'état déshydraté dans une chambre (6) située du côté de l'aiguille, et dont le solvant est situé dans l'autre chambre (7), et dans lequel les deux chambres (6,7) sont séparées l'une de l'autre par un bouchon (4), qui, pour l'exécution du mélange intime de la substance avec le solvant, avant l'application, peut être amené dans la zone d'un by-pass (8) qui est prévu dans le cylindre (1) de la seringue et s'étend dans sa direction axiale et dont la longueur est supérieure à l'épaisseur axiale du bouchon (4), et dans lequel le bouchon (4) est déplacé, directement ou par l'intermédiaire du solvant, en direction de l'extrémité située du côté de l'aiguille, par l'intermédiaire du piston (3) de la seringue qui limite la seconde chambre (7) et est équipée d'une tige de piston (10), caractérisé en ce que, en position verticale alors que l'extrémité (5) de montage de l'aiguille est tournée vers le haut, on déplace tout d'abord le bouchon (4) à une vitesse limitée d'une manière conditionnée par le dispositif, en l'amenant dans la zone du by-pass (8), qu'on déplace ensuite le piston (3) de la seringue jusqu'à ce qu'il s'applique contre le bouchon (4), grâce à une libre mobilité de la tige de piston (10) jusqu'au niveau d'une butée avant (14.1) et que de ce fait le solvant est transféré dans la chambre (6) située du côté de l'aiguille, qu'ensuite, jusqu'à obtention de la dissolution complète de la substance, on rétracte à nouveau une ou plusieurs fois le piston (3) de la seringue jusqu'au niveau d'une butée arrière (13.1) de la tige de piston (10) et qu'ensuite à nouveau on le déplace jusqu'au niveau de la butée avant (14.1) de sorte que la substance active reflue à nouveau, ainsi que le solvant, depuis la chambre avant (6) dans la chambre arrière (7) en franchissant le by-pass (8), puis dans la chambre avant (6) et qu'enfin, après dissolution complète de la substance active, on desserre la butée avant (14') de la tige de piston (10), qui limite tout d'abord le déplacement du piston (3) de l'aiguille en direction de l'extrémité, située du côté de l'aiguille, du cylindre (1) de la seringue, pour l'application de la substance pharmaceutique.

2. Procédé suivant la revendication 1, caractérisé par le fait qu'on installe la canule (2) lorsque la tige de piston (10) est déplacée jusqu'au niveau de la butée arrière (13.1).

3. Seringue à usage médical pour la mise en oeuvre du procédé selon les revendications 1 et 2, comportant un cylindre (1) installé sur une extrémité prévue pour le montage d'une aiguille d'injection (2), et un piston (3) déplaçable dans le cylindre (1) de la seringue, ainsi qu'un bouchon (4), qui est disposé entre l'extrémité (5) de montage de l'aiguille et le piston (3) de la seringue et forme ainsi deux chambres (6,7) d'une seringue à deux chambres, et dans laquelle la surface enveloppe du cylindre (1) de la seringue comporte, dans la chambre (6) située du côté de l'aiguille, une partie élargie en coupe transversale, qui s'étend axialement et forme un by-pass (8) et au moyen de laquelle les deux chambres (6,7) sont réunies entre elles lorsque le bouchon est amené dans la zone (8) du by-pass (4), la seringue comportant également un élément d'amortissement (9) qui est utilisé comme frein pour le piston et est situé sur l'extrémité du cylindre tournée à l'opposé de l'extrémité (5) de montage de l'aiguille, caractérisée en ce que l'élément d'amortissement (9) limite la vitesse de déplacement de la tige de piston (10) jusqu'à ce que le bouchon (4) soit amené dans la zone du by-pass (8), et que la tige de piston (10) comporte, pour la limitation de la course de déplacement du piston (3) de la seringue entre sa position initiale contre l'extrémité du cylindre et sa position dans laquelle il est appliqué contre le bouchon (4) amené dans la zone du by-pass, une butée avant (14.1) et une butée arrière (13.1) pour un élément antagoniste prévu sur l'extrémité du cylindre, et que la butée avant (14.1), qui limite le déplacement du piston (3) de la seringue en direction de l'extrémité située du côté de l'aiguille, peut être desserrée.

4. Seringue selon la revendication 3, caractérisée en ce que l'élément d'amortissement (9) est formé par un appui-doigt (12), qui est monté sur un rebord en verre (11) présent sur le côté du cylindre (1) de la seringue, tourné à l'opposé de l'extrémité (5) de montage de l'aiguille, et est pourvu d'un taraudage qui est coaxial au cylindre (1) de la seringue et est prévu pour un manchon fileté (13) installé sur la tige (10) du piston, le taraudage constituant l'élément antagoniste, tandis que la face frontale, tournée à l'opposé du cylindre de la seringue, forme la butée arrière et que la longueur du manchon fileté (13) est dimensionnée de manière que sa face frontale, tournée à l'opposé du piston (3) de la seringue, sort librement du taraudage dès que le bouchon (4) est situé dans la zone médiane du by-pass (8).

5. Seringue selon la revendication 4, caractérisée en ce que la butée avant (14.1) est formée par la surface, tournée vers le premier manchon fileté (13), d'un second manchon fileté (14) qui correspond également au taraudage.

6. Seringue selon la revendication 4 ou 5, caractérisée en ce que la tige de piston (10) possède frontalement un boit fileté (15) pour un filetage du bouchon prévu dans le piston (3) de la seringue, auquel cas entre le bout fileté (15) et le premier manchon fileté (13) il est prévu un élément cylindrique de guidage (16), dont le diamètre est seulement légèrement inférieur au diamètre de base du taraudage.

7. Seringue selon les revendications 4 à 6, caractérisée en ce que l'appui-doigt (12) est formé par deux demi-coques, qui logent entre elles le rebord en verre (11) du cylindre (1) de la seringue et sont équipées d'éléments de liaison (17) qui s'encliquettent réciproquement, la demi-coque, située du côté de la canule, étant pourvue d'un logement qui entoure le cylindre (1) de la seringue, tandis que l'autre demi-coque est équipée du taraudage.

8. Seringue selon la revendication 7, caractérisée en ce que les deux demi-coques sont raccordées entre elles d'une manière articulée au niveau de leurs bords.
